# EUROPEAN PATENT APPLICATION

(11) **EP 2 036 976 A1**
(43) Date of publication of application: **18.03.2009**
(21) Application number: 07745429.6
(22) Date of filing: 15.06.2007
(51) Int. Cl.: C12M 1/00, C12N 5/06

(54) **MICROCHIP FOR CELL SAMPLING AND METHOD OF CELL SAMPLING**

(30) Priority: 16.06.2006 JP 2006168012
(71) Applicant: ABsize Inc., Osaka-shi, Osaka 530-0053 (JP)
(72) Inventor: SATO, Setsuya c/o Cybox Co., Ltd, Ibaraki-shi, Osaka 567-0085 (JP); YAMATO, Toshiyuki c/o Cybox Co., Ltd, Ibaraki-shi, Osaka 567-0085 (JP); HAYASHI, Katsuaki c/o Cybox Co., Ltd, Ibaraki-shi, Osaka 567-0085 (JP); MATSUMOTO, Yoshitaka c/o Cybox Co., Ltd, Ibaraki-shi, Osaka 567-0085 (JP); OH, Isamu c/o GRADUATE SCHOOL OF ENGINEERING, Suita-shi, Osaka 565-0871 (JP)
(74) Representative: Skuhra, Udo
(86) International application number: PCT/JP2007/062175
(87) International publication number: WO 2007/145342

(57) **Abstract**

[Problems] To provide a microchip for cell sorting to obtain the desired cell from a number of cells quickly and accurately by combining the advantages from both methods using the optical tweezer and the liquid supply to the micro channel.

[Means for solving problems] A microchip for sorting at least one cell including a base, the base comprising: a cell supply channel for supplying and flowing a liquid containing cells; a cell supply channel including an inlet port in liquid communication with a supply mechanism and a drain port in liquid communication with a drain mechanism; a cell sorting channel intersecting with the cell supply channel at their middle point, the cell sorting channel including an inlet port in liquid communication with a supply mechanism and a drain port in liquid communication with a drain mechanism.

## Description

### BACK GROUND OF THE INVENTION

### FIELD OF THE INVENTION

This invention relates to a microchip and method for cell sorting. Further, it relates to a method utilizing liquid pumping and optical tweezer both together to transfer cells during a sorting operation, and relates to a microchip for cell sorting used for this method.

### DESCRIPTION OF THE RELATED ART

An optical tweezer manipulation is known as a method of cell transfer in a non contact manner by trapping and scanning a cell with a laser beam radiation pressure (e.g. Patent Reference 1).

This method advantageously provides accurate and free cell transfer but is not efficient due to slow transfer speed which results in a lot of time when transferring numbers of cells or long distances.

Another non-contact method for cell transfer is sending a liquid to a micro channel with micro pump and so on (e.g. Patent Reference 2).

This method has an advantage in transferring the numbers of cells quickly at once. However, it has some difficulties in controlling the cell numbers to be transferred and selecting the cells. Further, it is unsuitable for short and accurate movement.

As described above, both of the methods have the problems respectively.

The same problem has been found in the cell sorting manipulation to obtain desired cells from a number of cells. In other words, it is inefficient to sort the desired cells with the optical tweezer in capable of quick manipulation, while it is difficult to sort the desired cells accurately in the manipulation with sending the liquid to the micro channel.
[Patent Reference 1]
   Japanese Patent Publication 2001-62792
[Patent Reference 2]
   Japanese Patent Publication 2003-274924

### DISCLOSURE OF THE INVENTION

### PROBLEMS TO BE SOLVED BY THE INVENTION

This invention is to solve the above-described problems. It further provides a method for cell sorting to obtain the desired cell from a number of cells quickly and accurately by combining the advantages from both methods using the optical tweezer and the liquid supply to the micro channel. It also provides a microchip for cell sorting for this method.

### MEANS FOR SOLVING THE PROBLEMS

The invention according to claim 1 relates to a microchip for sorting at least one cell including a base, the base comprising: a cell supply channel for supplying and flowing a liquid containing cells; a cell supply channel including an inlet port in liquid communication with a supply mechanism and a drain port in liquid communication with a drain mechanism; a cell sorting channel intersecting with the cell supply channel at their middle point, the cell sorting channel including an inlet port in liquid communication with a supply mechanism and a drain port in liquid communication with a drain mechanism.

The invention according to claim 1 relates the microchip for cell sorting wherein the cell sorting channel comprises a reservoir wider than the cell sorting channel between the middle point and the drain port for a temporal storage of the cells flowing to the drain port.

The invention according to claim 3 relates to a method for cell sorting at least one cell using the microchip described in claim 1 or 2, comprising: a first step of providing the cell supply channel with the liquid from the supply mechanism so that the liquid containing the cells flows through the supply channel,
a second step of stopping the liquid flow from the supply mechanism when a desired cell existing in the liquid arrives in a vicinity of the middle point, and
a third step of operating optical tweezer to transfer the desired cell into the cell sorting channel.

The invention according to claim 4 relates to a method further comprising: a step of providing the microchip described in claim 2, a step of repeating the first to the third steps until transferring a desired number of the desired cells to the cell sorting channel, a fourth step of operating the supply mechanism and the drain mechanism coupled to the cell supply channel to drain out all the cells in the cell supply channel except the number of the desired cells, a fifth step of sending the number of the desired cells to the reservoir using a liquid from the supply mechanism coupled to the cell sorting channel, and a sixth step of extracting the desired cell in the reservoir.

### EFFECT OF THE INVENTION

According to the invention of claim 1, the liquid containing the cells from the supply mechanism can flow through the cell supply channel quickly, and the cell sorting channel intersecting with the cell supply channel at their middle point. Therefore, sorting of the desired cell from the intersection to the cell sorting channel can be performed accurately with the optical tweezer after the liquid supply stops. Thus, quick and accurate sorting of the desired cell from the number of cells can be achieved.

According to the invention of claim 2, the cell sorting channel comprises a reservoir wider than the cell sorting channel for a temporal storage of the cells flowing to the drain port. The reservoir can store the desired cell sorted into the cell sorting channel. Thus, extraction of the desired cell from the reservoir with micropipette and so on can be easily performed.

According to the invention of claim 3, the desired cell can be sorted quickly and accurately from the number of cells supplied to the cell supply channel into the cell sorting channel. This is achieved by the following 3 steps: a first step of providing the cell supply channel with the liquid from the supply mechanism so that the liquid containing the cells flows through the supply channel quickly; a second step of stopping the desired cell in a vicinity of the middle point; and a third step of operating the optical tweezer to transfer the desired cell into the cell sorting channel.

According to the invention of claim 4, an unnecessary cell is prevented from flowing into the cell sorting channel so that only the desired cell can be surely sorted to be extracted. This is achieved by the following 3 steps: draining out all the cells in the cell supply channel except the desired cells; sending the desired cells to the reservoir; and extracting the desired cells in the reservoir.

### DESCRIPTION OF THE PREFERRED EMBODIMENTS

Hereinafter, preferred embodiments of a microchip and method for cell sorting according to the present invention will be described with reference to the drawings.

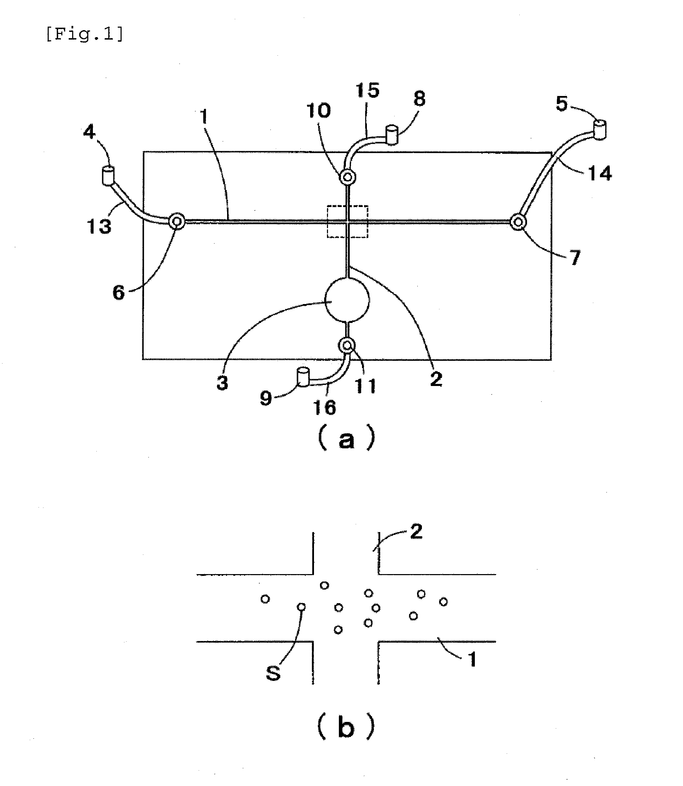

Fig. 1 is a top view of the microchip for cell sorting (hereinafter referred to as a microchip) of this invention. Fig.1(a) shows a overall view, and Fig.1(b) shows an enlarged view of a dashed square in (a). A cell is shown as circle shape designated by a character (S).

The microchip of this invention includes an approximately flat base formed with a synthetic resin with optical transparency such as a light curing resin, glass or their combination. The base comprises a cell supply channel 1, a cell sorting channel 2 and a reservoir 3.

The cell supply channel 1 for transferring cells delivered with a liquid from a supply mechanism is a hollow channel about 100µm in an inner diameter buried inside the base so that it does not appear on the outer surface of the base.

The cell supply channel 1 is formed with an inlet port 6 in liquid communication with a supply mechanism 4 via a tube 13 at its start point. It is also formed with a drain port 7 in liquid communication with a drain mechanism 5 via a tube 14 at its end point.

Similarly to the cell supply channel 1, the cell sorting channel 2 for sorting and transferring only the desired cells delivered with a liquid from a supply mechanism 4 is a hollow channel about 100µm in an inner diameter buried inside the base so that it does not appear on the outer surface of the base.

The cell sorting channel 2 includes an inlet port 10 in liquid communication with a supply mechanism 8 via a tube 15. It also includes a drain port 11 in liquid communication with a drain mechanism 9 via a tube 16 at its end point.

The known supply and drain mechanism, such as a micropump and a microsyringe, for supplying and draining the liquid in the micro channel can be applied to the above-described supply mechanism 4,8 and drain mechanism 5,9.

The cell sorting channel 2 intersects with the cell supply channel 1 at their middle point.

Their crossing angle is not limited to 90 degrees, although they cross at the right angle in the drawings.

The cell sorting channel 2 comprises a reservoir 3 between their intersection and the drain port 11 for a temporal storage of the cells flowing to the drain port 11.

The reservoir 3 is wider than the cell sorting channel 2 (for example, more than twice, preferably 5 times) . Thus, the flow of the liquid containing the cells via the cell sorting channel 2 from their intersection is decelerated at the reservoir 3. This prevents the liquid from flowing into the drain port 11.

Also, the reservoir 3 has an opening on the base surface so that the desired cell can be extracted therefrom with a micropipette and so on.

Next, the cell sorting method of this invention will be explained.

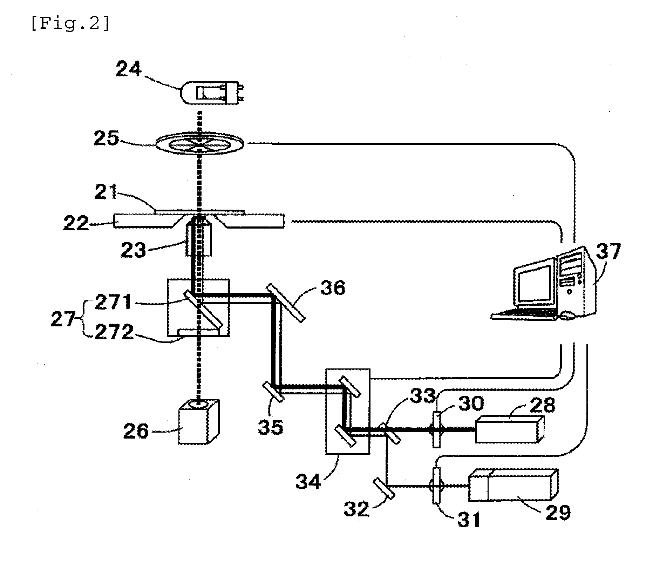

Fig. 2 is a schematic view showing one example of a device used in the cell sorting method of this invention. The device consists of an inverted microscope combined with a laser source and so on.

Now, a structure of this device will be explained.

The microscope includes an electric stage 22 capable of moving along an X-Y axis and supporting the microchip 21, an objective lens 23 placed directly below the electric stage 22 to focus a light from the laser source (to be described later) and guide the light to the microchip 21, a laser source lamp 24 with a halogen lamp placed vertically above the objective lens 23 to send the light to the microchip 21, an electric shutter 25 placed between the laser source lamp 24 and the microchip 21 to control a quantity of light from the laser source lamp 24 to the microchip 21, and an imaging device 26 such as CCD camera or CMOS camera which captures a transmission image of a visible light from the laser source lamp 24 passing the microchip 21.

Movement of the electric stage 22 and opening /closing operation of the electric shutter 25 are controlled by a control signal from a control software of a computer 37.

A mirror unit 27 comprising a dichroic mirror 271 and an absorbing filter 272 is placed vertically below the objective lens 23.

The dichroic mirror 271 changes a light path from the laser source toward to the objective lens 23. The dichroic mirror 271 also passes the light from the laser source lamp 24 and guides it to the imaging device 26.

The absorbing filter 272 passes only the visible light components selectively from light components with various wavelengths from the laser source lamp 24 after the dichroic mirror 271.

The laser source comprises a first laser source 28 which emits an IR laser and a second laser source 29 which emits a UV laser.

The IR laser from the first laser source 28 is used as a trapping laser to trap and control the cell, i.e., an optical tweezer. For example, YAG laser (wavelength of 1060nm), Nd:YLF laser (wavelength 1047nm), DPSS laser (wavelength of 1064nm) and so on can be used as the IR laser, but not limited to these lasers as long as it is controlled without any damages to cells.

The UV laser from the second laser source 29 is used as a cell fusion laser. However, the second laser source 29 is not always necessary for the method of the invention.

Electric shutters 30,31, dichroic mirrors 32,33,35,36 and electric mirror unit 34 are placed on an optical guiding path which leads the laser light from the first laser source 28 and the second laser source 29 to the above-described dichroic mirror 271 of the mirror unit 27.

Electric shutters 30,31 are placed in front of exit apertures of the first laser source 28 and the second laser source 29 respectively.

They can be opened and closed independently by the control signal from the computer 37. Thus the laser light from the first laser source 28 and the second laser source 29 can be led to the mirror unit 27 selectively via the electric mirror unit 34 and so on.

The dichroic mirror 33 is placed in front of the electric shutter 30, and the dichroic mirror 32 is placed in front of the electric shutter 31.

The dichroic mirror 32 changes laser light path from the second laser source 29 toward the dichroic mirror 33.

The dichroic mirror 33 passes the laser light from the first laser source 28 to the electric mirror unit 34. It also reflects the laser light from the second laser source 29 coming from dichroic mirror 32 and leads it to the electric mirror unit 34.

Therefore, the laser light from the first laser source 28 and the second laser source 29 are lead to the electric mirror unit 34 along the same path by the above-described dichroic mirrors 32,33.

The electric mirror unit 34 has two electric control mirrors which are controllable independently by the control signal from the computer 37. One mirror scans in the x-axis direction and the other in the y-axis direction when the scanning is performed in the microchip 21 on the electric stage 22.

A galvanometer mirror, a piezo-driven mirror, an actuator-driven mirror and so on are applicable to the electric control mirror.

The dichroic mirror 35 changes laser light path after the electric mirror unit 34 to the dichroic mirror 36.

The dichroic mirror 36 changes this laser light path toward the dichroic mirror 271 of the mirror unit 27.

The method for sorting a cell according to the present invention is accomplished by using the above- described device as below.

At first, the microchip 21 of the present invention described above is fixed on the electric stage 22 of an invert microscope. The supply mechanism 4,8 and the drain mechanism 5,9 such as a micro pump or a micro syringe are prepared as well.

Then, as shown in Fig. 1, the supply mechanism 4 is coupled to the inlet port 6 via the tube 13 and the drain mechanism 5 is connected to the drain port 7 via the tube 14. The supply mechanism 8 is coupled to the inlet port 10 via the tube 15 and the drain mechanism 9 is connected to the drain port 11 via the tube 16.

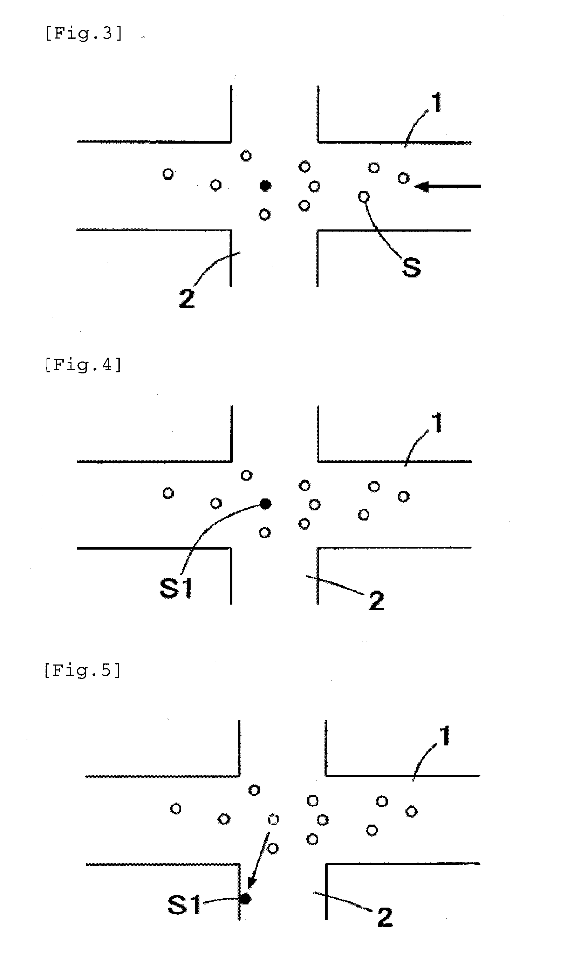

As the first step, the supply mechanism 4 sends the liquid containing the cells S to the cell supply channel 1 (Fig.3).

As the second step, the liquid flowing in the cell supply channel 1 is observed to confirm that a desired cell S1 is arrived at the intersection with the cell sorting channel 2, and then the liquid supply from the supply mechanism 4 is stopped (Fig.4).

As the third step, the desired cell S1 is transferred to the cell sorting channel 2 using the optical tweezer (Fig. 5). In this optical tweezer method, IR laser outputted from the first laser source 28 traps the cell S1 by controlling the electric mirror unit 34 to guide the laser beam to the cell S1 which then is moved to the cell sorting channel 2.

After transferring the cell S1 with the optical tweezer to the cell sorting channel 2, the first to the third steps are then repeated. In short, a fresh liquid containing the cells is sent to the cell supply channel 1 from the supply mechanism 4 to push out unnecessary remaining cells S in the intersection and supply fresh cells (Fig. 6), then when another desired cell S1 figured out among the cells in the fresh liquid arrives at the intersection, the supply mechanism 4 stops supplying the liquid (Fig. 7), and finally the desired cells S1 are moved to the cell sorting channel 2 with the optical tweezer (Fig. 8).

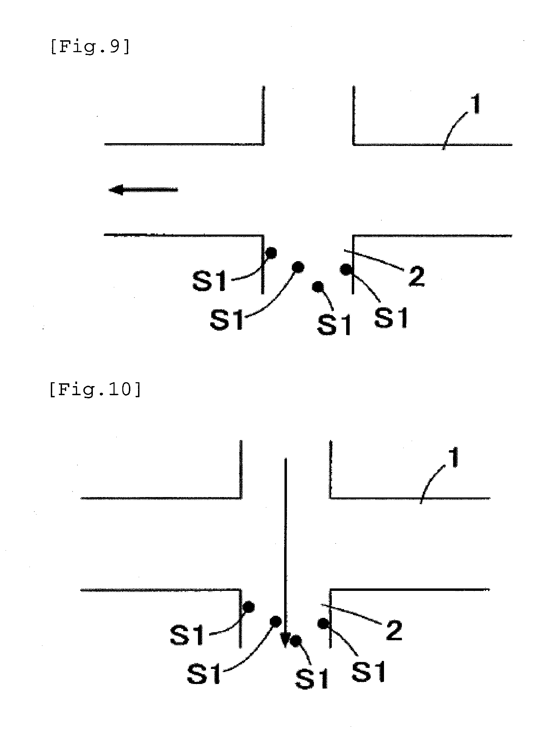

The first to the third steps are repeated until all the desired cells are transferred to the cell sorting channel 2, then as the fourth step, the operation of the supply mechanism 4 in liquid communication with the inlet port to send the liquid and the operation of the drain mechanism to drain out the liquid removes cells from the cell supply channel except the desired cells S1 (Fig. 9).

As the fifth step, the supply mechanism 8 connected to the cell sorting channel 2 supplies the liquid to send the desired cells S1 into the reservoir (Fig. 10).

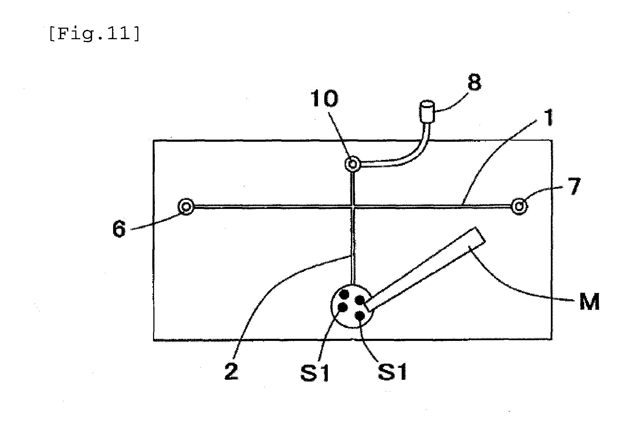

Finally as the sixth step, the desired cells S1 in the reservoir 3 extracted with a micro pipette (M) and so on (Fig. 11).

These above mentioned steps provide quick and accurate extraction of the desired cells delivered in the reservoir 3 via the cell sorting channel 2 from multiple cells supplied to the cell supply channel.

### Industrial Utilization

The present invention is preferably applicable to a life science technology to sort out the desired cells from multiple cells.

### Brief Description of Drawings

Fig. 1 is a schematic plane view of the microchip for sorting cells according to the present invention. Fig. 1(a) is an overall view and Fig. 1 (b) is an enlarged view in the dotted square in Fig.1 (a)
Fig.2 is a schematic view showing the device used for the method for sorting cells according to the present invention.
Fig. 3 shows the first step of the method for sorting cells according to the present invention.
Fig. 4 shows the second step of the method for sorting cells according to the present invention.
Fig. 5 shows the third step of the method for sorting cells according to the present invention.

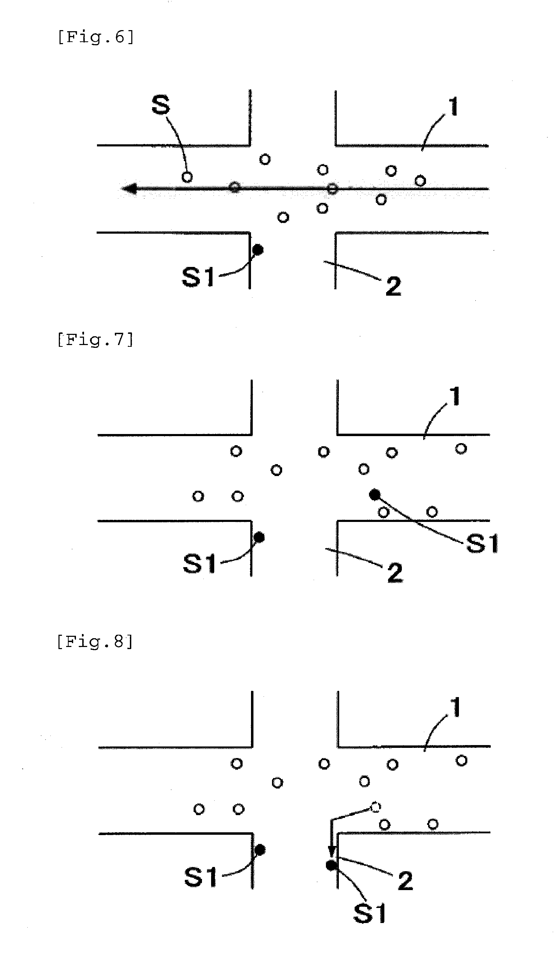

Fig. 6 shows the first step in the second operation of the method for sorting cells according to the present invention.
Fig. 7 shows the second step in the second operation of the method for sorting cells according to the present invention.
Fig. 8 shows the third step in the second operation of the method for sorting cells according to the present invention.
Fig. 9 shows the fourth step of the method for sorting cells according to the present invention.
Fig. 10 shows the fifth step of the method for sorting cells according to the present invention.
Fig. 11 shows the sixth step of the method for sorting cells according to the present invention.

### Explanation on the Numerals

- 1: Cell Supply Channel
- 2: Cell Sorting Channel
- 3: Reservoir
- 4: Supply Mechanism
- 5: Drain Mechanism
- 6: Inlet Port
- 7: Drain Port
- S: Cell
- S1: Desired Cell

## Claims

1. A microchip for sorting at least one cell including a base, the base comprising:
a cell supply channel for supplying and flowing a liquid containing cells;
a cell supply channel including an inlet port in liquid communication with a supply mechanism and a drain port in liquid communication with a drain mechanism;
a cell sorting channel intersecting with the cell supply channel at their middle point, the cell sorting channel including an inlet port in liquid communication with a supply mechanism and a drain port in liquid communication with a drain mechanism.

2. The microchip for cell sorting according to claim 1, wherein the cell sorting channel comprises a reservoir wider than the cell sorting channel between the middle point and the drain port for a temporal storage of the cells flowing to the drain port.

3. A method for cell sorting at least one cell using the microchip described in claim 1 or 2, comprising:
a first step of providing the cell supply channel with the liquid from the supply mechanism so that the liquid containing the cells flows through the supply channel,
a second step of stopping the liquid flow from the supply mechanism when a desired cell existing in the liquid arrives in a vicinity of the middle point, and
a third step of operating optical tweezers to transfer the desired cell into the cell sorting channel.

4. The method according to claim 3 further comprising:
a step of providing the microchip described in claim 2,
a step of repeating the first to the third steps until transferring a desired number of the desired cells to the cell sorting channel,
a forth step of operating the supply mechanism and the drain mechanism coupled to the cell supply channel to drain out all the cells in the cell supply channel except the number of the desired cells,
a fifth step of sending the number of the desired cells to the reservoir using a liquid from the supply mechanism coupled to the cell sorting channel, and
a sixth step of extracting the desired cell in the reservoir.
